# EUROPEAN PATENT SPECIFICATION

(11) **EP 0 634 173 B1**
(45) Date of publication and mention of the grant of the patent: **16.09.1998**
(21) Application number: 94305104.5
(22) Date of filing: 13.07.1994
(51) Int. Cl.: A61K 31/59

(54) **24-epi-1-alpha-hydroxyvitamin D2 for treating osteoporosis**
24-Epi-1alpha-Hydroxyvitamin D2 zur Behandlung von Osteoporosis
24-Epi-1alpha-hydroxyvitamine D2 pour le traitement de l'ostéoporosis

(30) Priority: 13.07.1993 US 91223
(43) Date of publication of application: 18.01.1995
(73) Proprietor: WISCONSIN ALUMNI RESEARCH FOUNDATION, Madison, WI 53705 (US)
(72) Inventor: Deluca, Hector F., Deerfield, Wisconsin 53531 (US)
(74) Representative: Ellis-Jones, Patrick George Armine

(56) References cited:
- EP-A- 0 386 793
- WO-A-90/01321
- US-A- 4 588 716

## Description

### Background of the Invention

This invention relates to a use for treating or for preventing the depletion of calcium from bones as a result of osteoporosis.

More specifically, this invention relates to a use for treating or preventing various known forms of osteoporosis, e.g. postmenopausal, senile and steroid-induced osteoporosis, one of the characteristics of which is the loss of bone mass.

It is well known that females at the time of menopause suffer a marked loss of bone mass giving rise ultimately to osteopenia, which in turn gives rise to spontaneous crush fractures of the vertebrae and fractures of the long bones. This disease is generally known as postmenopausal osteoporosis and presents a major medical problem, both in the United States and most other countries where the life-span of females reaches ages of at least 60 and 70 years. Generally, the disease which is often accompanied by bone pain and decreased physical activity, is diagnosed by one or two vertebral crush fractures with evidence of diminished bone mass. It is known that this disease is accompanied by diminished ability to absorb calcium, decreased levels of sex hormones, especially estrogen and androgen, and a negative calcium balance.

Similar symptoms of bone loss characterize senile osteoporosis and steroid-induced osteoporosis, the latter being a recognized result of long term glucocorticoid (cortico-steroid) therapy for certain disease states.

Methods for treating the disease have varied considerably but to date no totally satisfactory treatment is yet known. A conventional treatment is to administer a calcium supplement to the patient. However, calcium supplementation by itself has not been successful in preventing or curing the disease. Another conventional treatment is the injection of sex hormones, especially estrogen, which has been reported to be effective in preventing the rapid loss of bone mass experienced in postmenopausal women. This technique, however, has been complicated by the fear of its possible carcinogenicity. Other treatments for which variable results have been reported, have included a combination of vitamin D in large doses, calcium and fluoride. The primary problem with this approach is that fluoride induces structurally unsound bone, called woven bone, and in addition, produces a number of side effects such as increased incidence of fractures and gastrointestinal reaction to the large amounts of fluoride administered. Another suggested method is to block bone resorption by injecting calcitonin or providing phosphonates.

U. S.-A- No. 4,225,596 suggests the use of various metabolites of vitamin D₃ for increasing calcium absorption and retention within the body of mammals displaying evidence of or having a physiological tendency toward loss of bone mass. The metabolites specifically named in that patent, i.e., 1α-hydroxyvitamin D₃, 1α-hydroxyvitamin D₂, 1α,25-dihydroxyvitamin D₃, 1α,25-dihydroxyvitamin D₂ and 1,24,25-trihydroxyvitamin D₃, although capable of the activity described and claimed in that patent, are also characterized by the disadvantage of causing hypercalcemia, especially if used with the conventional calcium supplement treatment. `Therefore, use of these compounds to treat osteoporosis has not been widely accepted. U. S.-A- Nos. 3,833,622 and 3,901,928 respectively suggest using the hydrate of 25-hydroxyvitamin D₃ and 1α-hydroxyvitamin D₃ for treatment of osteoporosis in a general expression of utility for those compounds. It is well known that both of those compounds express traditional vitamin D-like activity, including the danger of hypercalcemia.

U. S.-A- No. 4,588,716 also suggests the use of 1α,25-dihydroxy-24-epi-vitamin D₂ to treat bone disorders characterized by the loss of bone mass, such as osteoporosis. Although this compound expresses some of the vitamin D-like characteristics affecting calcium metabolism such as increasing intestinal calcium transport and stimulating the mineralization of new bone, it has the advantage of minimal effectiveness in mobilizing calcium from bone. The 1α,25-dihydroxy-24-epi-vitamin D₂ compound may be administered alone or in combination with a bone mobilization-inducing compound such as a hormone or vitamin D compound such as 1α-hydroxyvitamin D₃ or -D₂, or 1α,25-dihydroxyvitamin D₃ or -D₂.

### Summary of the Invention

It has now been found that the loss of bone mass, which is characteristic of osteoporosis may be effectively treated by the administration of a 1α-hydroxylated vitamin D₂ compound in sufficient amounts to increase bone mass. More specifically, this treatment for increasing bone mass and stimulating bone formation uses an effective amount of 24-epi-1α-hydroxyvitamin D₂. The above compound may be administered alone or in combination with other pharmaceutically acceptable agents. Dosages of from not less than about 0.5 µg/day to not more than about 15 µg/day of the individual compound per se, or in combinations, are generally effective. This use has the distinct advantage that it will restore bone mass due to the insignificant bone mobilization activity of this compound and further this compound advantageously will not cause hypercalcemia even if the compound is administered continuously on a daily basis, as long as the appropriate compound dosages are used, it being understood that the dosage levels will be adjusted dependent on the response of the subject as monitored by methods known to those skilled in the art.

Thus the present invention provides the use of 24-epi-1α-hydroxyvitamin D₂ for the preparation of a composition for increasing bone mass and stimulating bone formation.

The administration of the indicated dosages of 24-epi-1α-hydroxyvitamin D₂ is effective in restoring or maintaining bone mass, and thus this use can be for the treatment or prevention of various forms of osteoporosis such as postmenopausal osteoporosis, senile osteoporosis and steroid-induced osteoporosis. It will be evident that the use will find ready application for the prevention or treatment of disease states other than those named, in which the loss of bone mass is an indication.

### Disclosure of the Invention

The vitamin D compound useful in the present invention is 24-epi-1α-hydroxyvitamin D₂. The above compound may be administered alone or in combination with other pharmaceutically acceptable agents. 24-epi-1α-hydroxyvitamin D₂ is characterized by the following structural formula

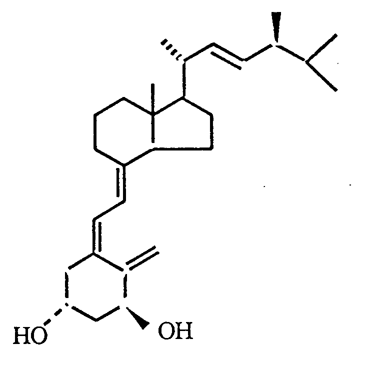

Reference is made to DeLuca et al U. S.-A-4,973,584 for a disclosure of the structure of 24-epi-1α-hydroxyvitamin D₂, a method of making 24-epi-1α-hydroxyvitamin D₂ and the biological activity of 24-epi-1α-hydroxyvitamin D₂.

This document also discloses that the compound promotes intestinal calcium absorption without significant calcium mobilisation from the bone. It also says that the compound promotes the calcification of bone.

The vitamin D compound can be readily formulated as sterile parenteral solutions by injection or intravenously, or by alimentary canal as oral dosages, or trans-dermally, or by suppository. Doses of from about 0.5 micrograms to about 15 micrograms per day of 24-epi-1α-hydroxyvitamin D₂ compound per se, or in combination with other 1α-hydroxylated vitamin D compounds, the proportions of each of the compounds in the combination being dependent upon the particular disease state being addressed and the degree of bone mineralization and/or bone mobilization desired, are generally effective. In all cases sufficient amounts of the compound should be used to restore or increase bone mass. Amounts in excess of about 15 micrograms per day of 24-epi-1α-hydroxyvitamin D₂ or the combination of that compound with other 1α-hydroxylated vitamin D compounds, are generally unnecessary to achieve the desired results, may result in hypercalcemia, and may not be an economically sound practice. In practice the higher doses are used where therapeutic treatment of a disease state is the desired end while the lower doses are generally used for prophylactic purposes, it being understood that the specific dosage administered in any given case will be adjusted in accordance with the specific compounds being administered, the disease to be treated, the condition of the subject and the other relevant medical facts that may modify the activity of the drug or the response of the subject, as is well known by those skilled in the art. For example, to be effective, 24-epi-1α-hydroxyvitamin D₂ is preferably administered in a dosage range of 0.5-15 µg/day. In general, either a single daily dose or divided daily dosages may be employed, as is well known in the art.

Dosage forms of the various compounds can be prepared by combining them with non-toxic pharmaceutically acceptable carriers to make either immediate release or slow release formulations, as is well known in the art. Such carriers may be either solid or liquid such as, for example, corn starch, lactose, sucrose, peanut oil, olive oil, sesame oil and propylene glycol. If a solid carrier is used the dosage form of the compounds may be tablets, capsules, powders, troches or lozenges. If a liquid carrier is used, soft gelatin capsules, or syrup or liquid suspensions, emulsions or solutions may be the dosage form. The dosage forms may also contain adjuvants, such as preserving, stabilizing, wetting or emulsifying agents, solution promoters, etc. They may also contain other therapeutically valuable substances.

The following Examples will serve to illustrate the efficacy of the compound and its suitability for the prevention or treatment of osteoporosis by increasing bone mass and stimulating bone formation. Examples 1 and 2 which are disclosed in US-A-4973584 and included for completeness.

### EXAMPLE 1

This example illustrates that administration of 24-epi-1α-hydroxyvitamin D₂ at the recommended dosages will not mobilize calcium from bone whereas the closely related compound 1α-hydroxyvitamin D₂ is strongly active in this regard. In animals on a low calcium diet, the elevation of serum calcium reflects the mobilization of calcium from bone.

1α-hydroxy-24-epi-vitamin D₂ was tested in the vitamin D-deficient rat. These tests indicate that 1α-hydroxy-24-epi-vitamin D₂ has a biological activity spectrum that is distinctly different from that of the previously known 1α-hydroxyvitamin D₂. In Table 1 below, representative assay results are given. These include tests of intestinal calcium transport activity ("S/M ratios"), and of bone mineral mobilization as reflected by serum calcium levels. These assays were conducted according to standard procedures (see e.g., U. S. Patent 4,588,716). The rats used in these assays were made vitamin D-deficient by maintenance on a vitamin D-free, low calcium diet (0.02% Ca, 0.37% P) for 3-1/2 weeks. They received the test compounds (or vehicle alone, i.e. -D control group) 20 h prior to sacrifice.

The data of Table 1 show that 1α-hydroxy-24-epi-vitamin D₂ exhibits high activity in stimulating intestinal calcium transport being essentially equivalent in this activity to the known 1α-hydroxyvitamin D₂. In contrast, 1α-hydroxy-24-epi-vitamin D₂ exhibits no activity in mobilizing calcium from bone. Thus 1α-hydroxy-24-epi-vitamin D₂, although structurally closely related to the known 1α-hydroxyvitamin D₂, exhibits a remarkably different activity profile. In stimulating the absorption of calcium, but not its liberation from bone, 1α-hydroxy-24-epi-vitamin D₂, is highly suitable as a therapeutic agent for the prevention or treatment of physiological conditions characterized by the loss of bone mass. Since 1α-hydroxy-24-epi-vitamin D₂ exhibits specificity of action, it is suitably administered alone, in situations where only calcium transport stimulation is desired, or together with graded doses of another active vitamin D compound--e.g. 1α-hydroxyvitamin D₂ or D₃, or 1α,25-dihydroxyvitamin D₃--in situations where some degree of bone mineral mobilization (together with calcium transport stimulation) is found to be advantageous.

**TABLE 1**

| Intestinal Calcium Transport and Bone Mobilization Activity of 1α-Hydroxyvitamin D₂ and 1α-Hydroxy-24-Epi-Vitamin D₂ | | | |
|---|---|---|---|
| | Amount (pmol) | Ca Transport S/M Ratio | Bone Mobilization Serum Ca mg % |
| -D (Control) | 0 | 2.5 ± 0.35 | 3.7 ± 0.20 |
| 1α-Hydroxy-24-epi-vitamin D₂ | 325 | 4.3 ± 0.42^{a} | 3.9 ± 0.39^{b} |
| | 650 | 4.4 ± 0.70^{a} | 4.1 ± 0.23^{b} |
| 1α-Hydroxy-vitamin D₂ | 325 | 5.4 ± 0.37^{a} | 5.3 ± 0.15^{a} |

| | | | |
|---|---|---|---|
| ^{a} Significant difference compared to respective control groups, p<0.001. | | | |
| ^{b} No significant difference compared to control. | | | |

### EXAMPLE 2

The results of Table 1 demonstrate that 1α-hydroxy-24-epi-vitamin D₂ is distuiguishable from 1α-hydroxyvitamin D₂ both structurally and in terms of its lack of activity in mobilizing calcium from bone. The results of Table 1, however, also demonstrate that, in terms of its calcemic action, 1α-hydroxy-24-epi-vitamin D₂ exhibits a biological activity spectrum similar to that of the known 1α,25-dihydroxy-24-epi-vitamin D₂. However, further tests showed that 1α-hydroxy-24-epi-vitamin D₂ is quite different from the known 1α,25-dihydroxy-24-epi-vitamin D₂ derivative in its activity in inducing the differentiation of malignant cells to normal monocytemacrophages. Differentiation activity was assayed using human leukemia cells (HL-60 cells), according to two standard tests, namely the nitroblue tetrazolium reduction (NBT-reduction) and the phagocytosis assays, and as shown in Table 2, 1α-hydroxy-24-epi-vitamin D₂ was compared against 1α,25-dihydroxyvitamin D₃ (a highly potent differentiation agent) and 1α,25-dihydroxy-24-epi-vitamin D₂.

The assays were conducted as described by Ostrem et al (J. Biol. Chem. 262, 14164-14171, 1987), and by DeLuca et al (U. S. Patent 4,717,721). The results given in Table 2 demonstrate that 1α,25-dihydroxyvitamin D₃ has, as expected, remarkable HL-60 cell differentiation activity. Even at doses as low as 10⁻⁸M, this compound produced approximately 64-67% differentiation in the 4-day trial period in both the NBT-reduction and the phagocytosis assay. 1α,25-dihydroxy-24-epi-vitamin D₂ is somewhat less active (about 5 times less active than the 1α,25-dihydroxyvitamin D₃ standard), but also shows very potent activity in this system, e.g. better than 60% differentiation at 5 x 10⁻⁸M and 80% differentiation at a concentration of 10⁻⁷M. In contrast, 1α-hydroxy-24-epi-vitamin D₂ possesses little or no cell differentiation activity. At best, only 16-20% differentiation was observed at a concentration of 10⁻⁷ M, and at a concentration of 1-2 x 10⁻⁸M, where the 1α,25-dihydroxy-24-epi-vitamin D₂ compound shows 40-50% differentiation, 1α-hydroxy-24-epi-vitamin D₂ does not elicit a significant differentiation response. Thus, 1α-hydroxy-24-epi-vitamin D₂ has little or no activity in promoting differentiation of promyelocytes to monocytes. These results show a marked biological difference between the present compound and the previously produced 1,25-dihydroxy-24-epi-vitamin D₂.

| Activity of 1α-Hydroxy-24-Epi-Vitamin D₂ in HL-60 Cell Differentiation | | | |
|---|---|---|---|
| Compound | Concentration (M) | % Differentiation | |
| | | NBT Reduction | Phagocytosis |
| 1α,25-Dihydroxy-vitamin D₃ | 1 x 10⁻⁷ | 87 ± 2 | 89 ± 3 |
| | 1 x 10⁻⁸ | 64 ± 2 | 67 ± 3 |
| 1α,25-Dihydroxy-24-epi-vitamin D₂ | 1 x 10⁻⁷ | 80 ± 3 | 81 ± 3 |
| | 5 x 10⁻⁸ | 64 ± 3 | 62 ± 3 |
| | 2 x 10⁻⁸ | 48 ± 3 | 49 ± 2 |
| | 1 x 10⁻⁸ | 39 ± 3 | 40 ± 3 |
| 1α-Hydroxy-24-epi-vitamin D₂ | 1 x 10⁻⁷ | 22 ± 2 | 16 ± 2 |
| | 5 x 10⁻⁸ | 14 ± 2 | 9 ± 1 |
| | 2 x 10⁻⁸ | 6 ± 2 | 6 ± 3 |
| | 1 x 10⁻⁸ | 4 ± 2 | 4 ± 2 |

Thus the preceding assays demonstrate that 1α-hydroxy-24-epi-vitamin D₂ exhibits a distinct and unique spectrum of activities--namely high potency in stimulating calcium transport, no activity in mobilizing calcium from bone, and little, if any, differentiation activity--which clearly distinguishes the compound from those of the prior art.

### EXAMPLE 3

This example illustrates that the compound used in the present invention is effective for restoring bone mass in rats made osteoporotic by ovariectomy.

In the experiment, female rats, between 5-7 weeks, were obtained from Sprague-Dawley and placed on a 0.23% calcium, 0.3% phosphorus diet together with normal levels of vitamin D₃ (75 IU/week). At 13 weeks, the animals were either sham-operated or oophorectomized. They continued on the same diet for an additional 16 weeks. Ouring the last 20 days, the animals received the indicated dose of either 1α-hydroxyvitamin D₂ or 24-epi-1α-hydroxyvitamin D₂, in propylene glycol, delivered each day by Alzet osmotic minipump for a period of 20 days. The animals were killed and their femurs were used to determine total bone ash and percent ash. The results are provided in Table 3. There were 6 animals in each group and the data are expressed as average ± standard error of the mean. Oophorectomy results in a lack of estrogen reminiscent of the menopausal state. This was accompanied by a loss of total bone mass, as shown by a drop of total femur ash to 322 mg from 387 mg. Note that at the same time, the percent ash remained the same, illustrating that total bone mass is what was lost. By providing 1α-hydroxyvitamin D₂ for 20 days, some of that ash was restored as total bone mass at a value of 377 mg. The most striking restoration took place with the 24-epi-1α-hydroxy-vitamin D₂ at 325 pmol/day, resulting in 400 mg of ash per femur. The animals thus actually increased their total bone mass above original sham-operated levels as a result of the administration of 24-epi-1α-hydroxyvitamin D₂.

This remarkable ability of 24-epi-1α-hydroxyvitamin D₂ to restore bone mass is superior to that of 1α-hydroxyvitamin D₂, the use of which is described in WO 90/01321 and which up to the present time appeared to be a superior compound in increasing bone mass of oophorectomized rats. Previously, 1α,25-dihydroxyvitamin D₃ and 1α-hydroxyvitamin D₃ had also been tested. These two compounds, although they restored some bone mass, were clearly not as effective as the compound of the present study.

Based on these results, 24-epi-1α-hydroxyvitamin D₂ should be, by far, the most effective vitamin D compound known today in treating postmenopausal and senile osteoporosis. Certainly it ranks as the most promising of the vitamin D compounds that are anticipated for treatment of this bone loss disorder.

**TABLE 3**

| RESTORATION OF BONE MASS BY 24-EPI-1α-OH-D₂ | | |
|---|---|---|
| Group | Total Femur Ash (mg ± SEM) | Ash (% ± SEM) |
| Sham-operated | 387 ± 16^{a} | 59.6 ± 0.1 |
| Oophorectomy (00X) | 322 ± 9.4^{b} | 58.5 ± 0.09 |
| 00X, 1α-OH-D₂ 325 pmol/day for 20 days | 377 ± 15.2^{c} | 58.6 ± 0.55 |
| 00X, 24-epi-1α-OH-D₂ 325 pmol/day for 20 days | 400 ± 16.4^{d} | 60.5 ± 0.5 |

| | | |
|---|---|---|
| ^{a} from ^{b}, p<0.005 | | |
| ^{c} from ^{b}, p<0.05 | | |
| ^{d} from ^{b}, p<0.005 ^{d} from ^{c}, p<0.05 | | |

## Claims

1. The use of 24-epi-1α-hydroxyvitamin D₂ for the preparation of a composition for increasing bone mass and stimulating bone formation.

2. The use according to claim 1 for the preparation of a composition for treating postmenopausal osteoporosis.

3. The use according to claim 1 for the preparation of a composition for treating senile osteoporosis.

4. The use according to claim 1 for the preparation of a composition for treating steroid-induced osteoporosis.

5. The use according to claim 1 for the preparation of a composition to be administered to women prior to the onset of menopause.

6. The use according to any one of the preceding claims wherein the vitamin D₂ compound is administered in an amount from about 0.5 micrograms to about 15 micrograms per day.

7. The use according to any one of the preceding claims wherein the vitamin D₂ compound, in solution in a liquid vehicle ingestible by and nontoxic to said patient, is administered orally in encapsulated form.

8. The use according to any one of claims 1 to 6 wherein the vitamin D₂ compound is administered in a slow release formulation.

9. The use according to any one of claims 1 to 7 wherein the vitamin D₂ compound is administered daily in divided dosages.

## Patentansprüche

1. Verwendung von 24-epi-1α-Hydroxyvitamin D₂ zur Herstellung einer Zusammensetzung zur Erhöhung der Knochenmasse und Stimulierung der Knochenbildung

2. Verwendung nach Anspruch 1 zur Herstellung einer Zusammensetzung zur Behandlung von postmenopausaler Osteoporose.

3. Verwendung nach Anspruch 1 zur Herstellung einer Zusammensetzung zur Behandlung von Altersosteoporose.

4. Verwendung nach Anspruch 1 zur Herstellung einer Zusammensetzung zur Behandlung von Steroid-induzierter Osteoporose.

5. Verwendung nach Anspruch 1 zur Herstellung einer Zusammensetzung, die Frauen vor Beginn der Menopause verabreicht wird.

6. Verwendung nach einem der vorhergehenden Ansprüche, dadurch gekennzeichnet, daß die Vitamin D₂-Verbindung in einer Menge von ca. 0,5 µg bis ca. 15 µg pro Tag verabreicht wird.

7. Verwendung nach einen der vorhergehenden Ansprüche, dadurch gekennzeichnet, daß die Vitamin D₂-Verbindung in Lösung in einem flüssigen Träger, der von dem Patienten verdaubar und für den Patienten nicht toxisch ist, oral in verkapselter Form verabreicht wird.

8. Verwendung nach einem der Ansprüche 1 bis 6, dadurch gekennzeichnet, daß die Vitamin D₂-Verbindung in einer Formulierung zur langsamen Freigabe verabreicht wird.

9. Verwendung nach einem der Ansprüche 1 bis 7, dadurch gekennzeichnet, daß die Vitamin D₂-Verbindung täglich in fraktionierten Dosierungen verabreicht wird.

## Revendications

1. Utilisation de la 24-épi-1α-hydroxyvitamine D₂ pour la préparation d'une composition destinée à augmenter la masse osseuse et à à stimuler la formation osseuse.

2. Utilisation selon la revendication 1 pour la préparation d'une composition destinée au traitement de l'ostéoporose post-ménopausique.

3. Utilisation selon la revendication 1 pour la préparation d'une composition destinée au traitement de l'ostéoporose sénile.

4. Utilisation selon la revendication 1 pour la préparation d'une composition destinée au traitement de l'ostéoporose induite par les stéroïdes.

5. Utilisation selon la revendication 1 pour la préparation d'une composition qui sera administrée à des femmes avant le début de la ménopause.

6. Utilisation selon l'une quelconque des revendications précédentes dans laquelle la vitamine D₂ est administrée à raison d'environ 0,5 µg à environ 15 µg par jour.

7. Utilisation selon l'une quelconque des revendications précédentes dans laquelle la vitamine D₂, en solution dans un véhicule liquide pouvant être ingéré par le patient et non toxique pour celui-ci, est administrée par voie orale sous une forme encapsulée.

8. Utilisation selon l'une quelconque des revendications 1 à 6, dans laquelle la vitamine D₂ est administrée dans une formulation à libération prolongée.

9. Utilisation selon l'une quelconque des revendications 1 à 7 dans laquelle la vitamine D₂ est administrée quotidiennement en plusieurs prises.
